# EUROPEAN PATENT SPECIFICATION

(11) **EP 1 341 810 B1**
(45) Date of publication and mention of the grant of the patent: **14.01.2009**
(21) Application number: 01985449.6
(22) Date of filing: 13.12.2001
(51) Int. Cl.: C07K 14/295, C12Q 1/68, G01N 33/68, A61K 39/118, A61P 15/00, C07K 16/12, G01N 33/571, C12N 15/74

(54) **SECRETED CHLAMYDIA POLYPEPTIDES AND METHOD FOR IDENTIFYING SUCH POLYPEPTIDES BY THEIR SECRETION BY A TYPE III SECRETION PATHWAY OF A GRAM-NEGATIVE BACTERIA.**
SEKRETIERTE CHLAMYDIA POLYPEPTIDE UND VERFAHREN ZUR IDENTIFIZIERUNG DAVON ÜBER IHRE SEKRETION DURCH GRAMNEGATIVEN BAKTERIENTYP III SEKRETIONSWEG
METHODE D'IDENTIFICATION DE POLYPEPTIDES DE CHLAMYDIA SECRETES EN UTILSANT UN SYSTEM DE SECRETION DE TYPE III D'UNE BACTERIE GRAM-NEGATIVE

(30) Priority: 14.12.2000 US 255118 P
(43) Date of publication of application: 10.09.2003
(73) Proprietor: INSTITUT PASTEUR, 75724 Paris Cédex 15 (FR); CENTRE NATIONAL DE LA RECHERCHE SCIENTIFIQUE (CNRS), 75794 Paris Cedex 16 (FR); INSTITUT NATIONAL DE LA SANTE ET DE LA RECHERCHE MEDICALE (INSERM), 75013 Paris (FR)
(72) Inventor: SUBTIL, Agathe, F-75013 Paris (FR); PARSOT, Claude, F-75015 Paris (FR); DAUTRY-VARSAT, Alice, F-75014 Paris (FR)
(74) Representative: Vialle-Presles, Marie José
(86) International application number: PCT/IB2001/002808
(87) International publication number: WO 2002/048185

(56) References cited:
- WO-A-99/45136
- FIELDS K A ET AL: "Evidence for the secretion of Chlamydia trachomatis CopN by a type III secretion mechanism." MOLECULAR MICROBIOLOGY, vol. 38, no. 5, December 2000 (2000-12), pages 1048-1060, XP002211782 ISSN: 0950-382X
- HSIA R ET AL: "TYPE III SECRETION GENES IDENTIFY A PUTATIVE VIRULENCE LOCUS OF CHLAMYDIA" MOLECULAR MICROBIOLOGY, BLACKWELL SCIENTIFIC, OXFORD, GB, vol. 25, no. 2, 1997, pages 351-359, XP008001280 ISSN: 0950-382X
- SUBTIL A ET AL: "TYPE III SECRETION SYSTEM IN CHLAMYDIA SPECIES: IDENTIFIED MEMBERS AND CANDIDATES" MICROBES AND INFECTION, ELSEVIER, PARIS, FR, vol. 2, no. 4, April 2000 (2000-04), pages 367-369, XP001065215 ISSN: 1286-4579
- FIELDS K A ET AL: "Analysis of type III secretion in Chlamydia trachomatis." ABSTRACTS OF THE GENERAL MEETING OF THE AMERICAN SOCIETY FOR, vol. 100, 2000, pages 270-271, XP008007055 100th General Meeting of the American Society for Microbiology;Los Angeles, California, USA; May 21-25, 2000, 2000 ISSN: 1060-2011
- ROSQVIST R ET AL: "FUNCTIONAL CONSERVATION OF THE SECRETION AND TRANSLOCATION MACHINERY FOR VIRULENCE PROTEINS OF YERSINIAE, SALMONELLAE AND SHIGELLAE" EMBO JOURNAL, OXFORD UNIVERSITY PRESS, SURREY, GB, vol. 14, no. 17, 1995, pages 4187-4195, XP002070536 ISSN: 0261-4189
- HERMANT D ET AL: "FUNCTIONAL CONSERVATION OF THE SALMONELLA AND SHIGELLA EFFECTORS OFENTRY INTO EPITHELIAL CELLS" MOLECULAR MICROBIOLOGY, BLACKWELL SCIENTIFIC, OXFORD, GB, vol. 17, no. 4, 1995, pages 781-789, XP001055768 ISSN: 0950-382X cited in the application
- SUBTIL AGATHE ET AL: "Secretion of predicted Inc proteins of Chlamydia pneumoniae by a heterologous type III machinery." MOLECULAR MICROBIOLOGY, vol. 39, no. 3, February 2001 (2001-02), pages 792-800, XP002211783 ISSN: 0950-382X
- JONES M A ET AL: "Secreted effector proteins of Salmonella dublin act in concert to induce enteritis" INFECTION AND IMMUNITY, AMERICAN SOCIETY FOR MICROBIOLOGY. WASHINGTON, US, vol. 66, no. 12, December 1998 (1998-12), pages 5799-5804, XP002116161 ISSN: 0019-9567 cited in the application

### SEQUENCE LISTING

<110> INSTITUT PASTEUR
   CNRS
   INSERM
   SUBTIL, Agathe
   PARSOT, Claude
   DAUTRY-VARSAT, Alice
<120> Secreted Chlamydia polypeptides and method for identifying such polypeptides by theirsecretion by a type III secretion pathway of a Gram-negative bacteria.
<130> S226PCT97
<140>
   <141>
<150> US N° 60/255,118
   <151> 2000-12-14
<160> 24
<170> PatentIn Ver. 2.1
<210> 1
   <211> 32
   <212> DNA
   <213> Artificial Sequence
<220>
   <223> Description of Artificial Sequence:primer
<400> 1
   tgacctcgag ttaacctatt aaggataaaa tt 32
<210> 2
   <211> 30
   <212> DNA
   <213> Artificial Sequence
<220>
   <223> Description of Artificial Sequence:primer
<400> 2
   gatcctcgag ttaatctatt tttagatagg 30
<210> 3
   <211> 30
   <212> DNA
   <213> Artificial Sequence
<220>
   <223> Description of Artificial Sequence:primer
<400> 3
   gatcctcgag ttaatctatt tttagatagg 30
<210> 4
   <211> 32
   <212> DNA
   <213> Artificial Sequence
<220>
   <223> Description of Artificial Sequence:primer
<400> 4
   gactaagctt gtaacctatt aaggataaaa tt 32
<210> 5
   <211> 32
   <212> DNA
   <213> Artificial Sequence
<220>
   <223> Description of Artificial Sequence:primer
<400> 5
   gactaagctt gtaatctatt tttagatagg aa 32
<210> 6
   <211> 32
   <212> DNA
   <213> Artificial Sequence
<220>
   <223> Description of Artificial Sequence:primer
<400> 6
   gactaagctt gtaagtaaaa aacacaaaaa at 32
<210> 7
   <211> 30
   <212> DNA
   <213> Artificial Sequence
<220>
   <223> Description of Artificial Sequence:primer
<400> 7
   gactaagctt gcatttgata attgcataaa 30
<210> 8
   <211> 27
   <212> DNA
   <213> Artificial Sequence
<220>
   <223> Description of Artificial Sequence:primer
<400> 8
   gactaagctt gaatacataa gctgttc 27
<210> 9
   <211> 29
   <212> DNA
   <213> Artificial Sequence
<220>
   <223> Description of Artificial Sequence:primer
<400> 9
   gactaagctt aaagtgtttg agatgaatt 29
<210> 10
   <211> 31
   <212> DNA
   <213> Artificial Sequence
<220>
   <223> Description of Artificial Sequence:primer
<400> 10
   gactaagctt attatataga cagattaaaa t 31
<210> 11
   <211> 31
   <212> DNA
   <213> Artificial Sequence
<220>
   <223> Description of Artificial Sequence:primer
<400> 11
   gactaagctt acaacaaatt aagatataat c 31
<210> 12
   <211> 32
   <212> DNA
   <213> Artificial Sequence
<220>
   <223> Description of Artificial Sequence:primer
<400> 12
   gactaagctt gtaaattgga gattgtagta gc 32
<210> 13
   <211> 30
   <212> DNA
   <213> Artificial Sequence
<220>
   <223> Description of Artificial Sequence:primer
<400> 13
   gactgaattc gttgctctat ctacgggtga 30
<210> 14
   <211> 30
   <212> DNA
   <213> Artificial Sequence
<220>
   <223> Description of Artificial Sequence:primer
<400> 14
   gactgaattc cttgttgtac ggacagtaat 30
<210> 15
   <211> 30
   <212> DNA
   <213> Artificial Sequence
<220>
   <223> Description of Artificial Sequence:primer
<400> 15
   ctaggaattc gattgaacag taacagatcc 30
<210> 16
   <211> 31
   <212> DNA
   <213> Artificial Sequence
<220>
   <223> Description of Artificial Sequence:primer
<400> 1-6
   gacttctaga aatgataacc ttttcaatga a 31
<210> 17
   <211> 31
   <212> DNA
   <213> Artificial Sequence
<220>
   <223> Description of Artificial Sequence:primer
<400> 17
   gacttctaga tccaggtttt tcggaagcag a 31
<210> 18
   <211> 31
   <212> DNA
   <213> Artificial Sequence
<220>
   <223> Description of Artificial Sequence:primer
<400> 18
   gacttctaga tatttgagct ggtacaacag g 31
<210> 19
   <211> 29
   <212> DNA
   <213> Artificial Sequence
<220>
   <223> Description of Artificial Sequence:primer
<400> 19
   gacttctaga cgctcgagaa taataaccc 29
<210> 20
   <211> 29
   <212> DNA
   <213> Artificial Sequence
<220>
   <223> Description of Artificial Sequence:primer
<400> 20
   gacttctaga aatgattagg taagcaatg 29
<210> 21
   <211> 29
   <212> DNA
   <213> Artificial Sequence
<220>
   <223> Description of Artificial Sequence:primer
<400> 21
   gacttctaga cgctcccaac cccagagtc 29
<210> 22
   <211> 31
   <212> DNA
   <213> Artificial Sequence
<220>
   <223> Description of Artificial Sequence:primer
<400> 22
   gacttctaga cttaaaaaat acccaggaac a 31
<210> 23
   <211> 31
   <212> DNA
   <213> Artificial Sequence
<220>
   <223> Description of Artificial Sequence:primer
<400> 23
   gacttctaga ttttattatt ttagcaatca c 31
<210> 24
   <211> 31
   <212> DNA
   <213> Artificial Sequence
<220>
   <223> Description of Artificial Sequence:primer
<400> 24
   gacttctaga aacaattgta tgattccatc c 31

## Description

The present invention relates to secreted *Chlamydia* polypeptides expressed by a Gram-negative bacterial strain and secreted by the type III secretion pathway of said bacterial strain.

Chlamydiae are Gram-negative bacteria that proliferate only within eukaryotic host-cells. The three species pathogenic to humans, *Chlamydia trachomatis, Chlamydia psittaci* and *Chlamydia pneumoniae*, cause a number of diseases, including trachoma, pelvic inflammatory disease, pneumonia and their sequelae (Gregory, D.W. and W. Schaffner. (1997). Psittacosis. Seminars in Respiratory Infections. 12: 7-11; Kuo, C.-C., L. Jackson, L. Campbell, and J. Grayston. (1995). Chlamydia pneumoniae (TWAR). Clin. Microbiol. Rev. 8: 451-61; Stamm, W.E. (1999). Chlamydia trachomatis infections: progress and problems. J. Infect. Dis. 179: S380-3).

During its cycle, *Chlamydia* adopts two distinct morphologies: a small infectious form, the elementary body (EB, 0.3 µm in diameter), and a larger replicative form, the reticulate body (RB, 1 µm in diameter) (Moulder, J.W. (1991). Interaction of Chlamydiae and host cells in vitro. Microbiol. Rev. 55: 143-90.). EBs are adapted for survival in the extracellular space: their outer membrane is made rigid by a network of disulfide bonds and the bacteria are metabolically inactive. They initiate infection by attaching to a suitable host cell, principally epithelial cells of the mucosa, and they are internalized by a mechanism resembling phagocytosis (Boleti, H., A. Benmerah, D. Ojcius, N. Cerf-Bensussan, and A. Dautry-Varsat. (1999). Chlamydia infection of epithelial cells expressing dynamin and Eps15 mutants: clathrin-independent entry into cells and dynamin-dependent productive growth. J. Cell. Sci. 112: 1487-1496). Within a few hours after internalization, EBs differentiate into replicative RBs, which proliferate in a growing vacuole and produce up to about a thousand orogeny. After 2 to 3 days of infection RBs differentiate back into EBs, which are delivered to the extracellular space, and a new infectious cycle can begin.

Throughout their cycle in the host cell, *Chlamydiae* remain in a membrane-bound compartment called an inclusion. Bacteria escape from host defense mechanisms by preventing fusion of the inclusion with acidic degradative compartments of host cells (Eissenberg, L.G., and P.B. Wyrick. (1981). Inhibition of phagolysosome fusion is limited to Chlamydia psittaci-laden vacuoles Infect. Immun. 32: 889-896; Friis, R.R. (1972). Interaction of L cells and Chlamydia psittaci: entry of the parasite and host responses to its development. J. Bacteriol. 110: 706-721; Heinzen, R.A., M.A. Scidmore, D.D. Rockey, and T. Hackstadt..(1996). Differential interaction with endocytic and exocytic pathways distinguish parasitophorous vacuoles of Coxiella burnetii and Chlamydia trachomatis. Infect. Immun. 64: 769-809; Schramm, N., C.R. Bagnell, and P.B. Wyrick. (1996). Vesicles containing Chlamydia trachomatis serovar L2 remain above pH 6 within HEC-1B cells. Infect. Immun. 64:1208-1214). In fact, *Chlamydiae* seem to exchange very little with the endocytic compartments (Taraska, T., D.M. Ward, R.S. Ajioka, P.B. Wyrick, S.R. Davis-Kaplan, C.H. Davis, and J. Kaplan. (1996). The late chlamydial inclusion membrane is not derived from the endocytic pathway and is relatively deficient in host proteins. Infect. Immun. 64: 3713-372; Van Ooij, C., G. Apodaca, and J. Engel. (1997). Characterization of the Chlamydia trachomatis vacuole and its interaction with the host endocytic pathway in HeLa cells. Infect. Immun. 65: 758-766). Yet, during their development cycle, the volume of the inclusions increases considerably, until they occupy a large portion of the cytosol. Part of the lipids-necessary for this growth come from the host cell (Hatch, G.M., and G. McClarty. (1998). Phospholipid composition of purified Chlamydia trachomatis mimics that of the eucaryotic host cell. Infection & Immunity. 66: 3727-35). Sphingomyelin, synthesized in the Golgi apparatus, is transported to the inclusion membrane and incorporated into bacteria (Hackstadt, T., M.A. Scidmore, and D.D. Rockey. (1995). Lipid metabolism in Chlamydia trachomatis-infected cells: directed trafficking of Golgi-derived sphingolipids to the chlamydial inclusion. Proc. Natl. Acad. Sci, USA. 92: 4877-4881). Surprisingly, no proteins of eukaryotic origin have been found associated with the inclusion. However, a number of procaryotic proteins have been localized on the membrane of the inclusion, by fluorescence microscopy using antibodies generated against a variety of chlamydial proteins (Bannantine, J.P., R.S. Griffiths, W. Viratyosin, W.J. Brown, and D.D. Rockey. (2000). A secondary structure motif predictive of protein localization to the chlamydial inclusion membrane. Cell. Microbiol. 2: 35-47.Bannantine, J.P., D.D. Rockey, and T. Hackstadt. (1998). Tandem genes of Chlamydia psittaci that encode proteins located to the inclusion membrane. Mol. Microbiol. 28: 1017-26; Rockey, D.D., R.A. Heinzen, and T. Hackstadt. (1995). Cloning and characterization of a Chlamydia psittaci gene cording for a protein localized in the inclusion membrane of infected cells. Mol. Microbiol. 15: 617-626; Scidmore-Carlson, M.A., E.I. Shaw, C.A. Dooley, E.R. Fischer, and T. Hackstadt. (1999). Identification and characterization of a Chlamydia trachomatis early operon encoding four novel inclusion membrane proteins. Mol. Microbiol. 33: 753-765). These proteins show no sequence similarities but have in common the presence of a large (40 to 70 amino acids) hydrophobic region and have therefore been grouped into a structural family called the Inc family. The three first Inc proteins identified, IncA, IncB and IncC, are probably major components of the inclusion since they are recognized by antisera from convalescent guinea pigs infected with *C*. *psittaci* Bannantine, J., W. Stamm, R. Suchland, and D. Rockey. (1998). Chlamydia trachomatis IncA is localized to the inclusion membrane and is recognized by antisera from infected humans and primates. Infect. Immun. 66: 6017-6021; Rockey, D.D., R.A. Heinzen, and T. Hackstadt. (1995). Cloning and characterization of a Chlamydia psittaci gene coding for a protein localized in the inclusion membrane of infected cells. Mol. Microbiol. 15: 617-626). They have homologs encoded by *C*. *trachomatis and C*. *pneumoniae* genomes, exhibiting about 20% global sequence identity and up to 50% sequence identity in the regions of highest similarity.

Inc proteins have been grouped into a family on two criteria: they have a large (larger than 40 residues) hydrophobic domain and they localize to the membrane of the inclusion in the host cell. The demonstration that several proteins encoded by the *C*. *trachomatis* genome and one protein from *C*. *pneumoniae* belong to this family was based on their localization to the membrane of the inclusion by immunofluorescence using specific antibodies (Bannantine, J., W. Stamm, R. Suchland, and D. Rockey. (1998). Chlamydia trachomatis IncA is localized to the inclusion membrane and is recognized by antisera from infected humans and primates. Infect. Immun. 66: 6017-6021;Bannantine, J.P., R.S. Griffiths, W. Viratyosin, W.J. Brown, and D.D. Rockey. (2000). A secondary structure motif predictive of protein localization to the chlamydial inclusion membrane. Cell. Microbiol. 2: 35-47; Bannantine, J.P., D.D. Rockey, and T. Hackstadt. (1998). Tandem genes of Chlamydia psittaci that encode proteins located to the inclusion membrane. Mol. Microbiol. 28: 1017-26; Rockey, D.D., R.A. Heinzen, and T. Hackstadt. (1995). Cloning and characterization of a Chlamydia psittaci gene coding for a protein localized in the inclusion membrane of infected cells. Mol. Microbiol. 15: 617-626.; Scidmore-Carlson, M.A., E.I. Shaw, C.A. Dooley, E.R. Fischer, and T. Hackstadt. (1999). Identification and characterization of a Chlamydia trachomatis early operon encoding four novel inclusion membrane proteins. Mol. Microbiol. 33: 753-765). However, the mechanism of their secretion and insertion into the membrane of the inclusion had not been investigated yet, due to the lack of genetic tools for members of *Chlamydia* spp.

How do chlamydial proteins reach the membrane of the inclusion? Four pathways of protein secretion (types I to IV) have been described in Gram-negative bacteria. Type III secretion machines allow for the translocation of bacterial effectors into the eukaryotic cell cytosol during bacterial contact with the cell surface (Hueck, C. (1998). Type III protein secretion systems in bacterial pathogens of animals and plants. Microbiol. Mol. Biol. Rev. 62: 379-433). The inventors hypothesized that *Chlamydiae* use type III secretion to insert proteins into the membrane of the inclusion (Subtil A., A. Blocker, and A. Dautry-Varsat. (2000). Type III secretion system in Chlamydia: identified members and candidates. Microbes Infect. 2: 367-369). Presence of genes encoding putative components of a type III secretion machinery in the *Chlamydia* genome suggested that Inc proteins might be secreted by a type III secretion pathway since type III secretion is used by a large number of Gram-negative pathogens for the insertion or the translocation of bacterial proteins into or through eukaryotic cell membranes. In the absence of genetic tools to manipulate the *Chlamydia* genome, it was difficult to test this hypothesis directly. *Shigella* uses a type III secretion system for entry into epithelial cells and for dissemination (Nhieu, G.T., and PJ. Sansonetti. (1999). Mechanism of Shigella entry into epithelial cells. Curr. Opin. Microbiol. 2: 51-5; Page, A.-L., H. Ohayon, P.J. Sansonetti, and C. Parsot. (1999). The secreted IpaB and IpaC invasins and their cytoplasmic chaperone IpgC are required for intercellular dissemination of Shigella flexneri. Cell. Microbiol. 1: 183-193; Schuch, R., R.C. Sandlin, and A.T. Maurelli. (1999). A system for identifying post-invasion functions of invasion genes: requirements for the Mxi-Spa type III secretion pathway of Shigella flexneri in intercellular dissemination, Mol. Microbiol. 34:675-689). The secretion signal involved in secretion of proteins by the type III secretion pathway is not known. However, it is located either within the first fifteen codons of the mRNA coding for the secreted proteins, or within the N-terminal part of the secreted protein (Anderson, D.M., and O. Schneewind. (1999). Type III machines of Gram-negative pathogens: injecting virulence factors into host cells and more. Curr. Opin. Microbiol. 2: 18-24). To test whether some chlamydial proteins, including members of the Inc family, were secreted via a type III secretion mechanism, hybrid proteins containing the N-terminal part of selected chlamydial proteins fused to a reporter protein were tested for their ability to be secreted by various strains of *S*. *flexneri*.

Here, the inventors have shown that several chlamydial proteins, including members of the Inc family and proteins selected for a hydropathic profile similar to that of Inc proteins, are secreted by the type III secretion machinery of *S*. *flexneri*.

Genes coding for the type III secretion system in *Shigella* are known (Hueck, C. (1998). Type III protein secretion systems in bacterial pathogens of animals and plants. Microbiol. Mol. Biol. Rev. 62: 379-433) and it has been hypothesized that Inc proteins may be secreted by a similar machinery.

The present inventors constructed chimeras by fusing the N-terminal part of these proteins with a reporter gene, the Cya protein of *Bordetella pertussis*, and expressed these chimeras in various strains of *Shigella flexneri*. The use of a reporter gene is well known by those of ordinary skill in the art. The reporter sequence, which codes for an easily detectable protein, is linked to a fragment of the DNA of interest. The localization of the hybrid protein depends on the results expected and the expression of the reporter gene gives information about the expression of the gene of interest and its expression product. The use of the *B*. *pertussis cya* gene as a reporter gene, chosen in the present invention, is reported by Jones *et al*., 1998 and Sory and Comelis, 1994 (Jones, M.A., M.W. Wood, P.B. Mullan, P.R. Watson, T.S. Wallis, and E.E. Galyov. (1998). Secreted effector proteins of Salmonella dublin act in concert to induce enteritis. Infection & Immunity. 66: 5799-804; Sory, M.P., and G.R. Cornelis. (1994). Translocation of a hybrid YopE-adenylate cyclase from Yersinia enterocolitica into HeLa cells. Mol. Microbiol. 14: 583-94).

Expression of heterologous proteins by use of type III machinery of *Shigella* has already been obtained (see "Functional conservation of the Salmonella and Shigella effectors of entry into epithelial cells" published in Molecular Microbiology (1995) 17 (4), 781-789, Hermant, Ménard, Arricau, Parsot and Popoff). However, as indicated in the title, this document relates to *Salmonella* proteins and is different from the present invention due to the phylogenic distance of *Chlamydia* from other organisms (see page 369, first column of Subtil, et al.; Microbes and Infection 2, 2000).

The present data that proteins secreted by *Chlamydia*, especially Inc proteins, are secreted by type III machinery and that they can be secreted in *Shigella* by *Shigella* type III machinery will allow development of various diagnostic and therapeutic tools.

Methods for screening inhibitors and activators of type III secretion machinery in Gram-negative bacteria, in *Shigella*, have already been disclosed in WO 99/58714.

A need continues to exist, however, for further elucidation of chlamydial polypeptides.

### BRIEF DESCRIPTION OF THE DRAWINGS

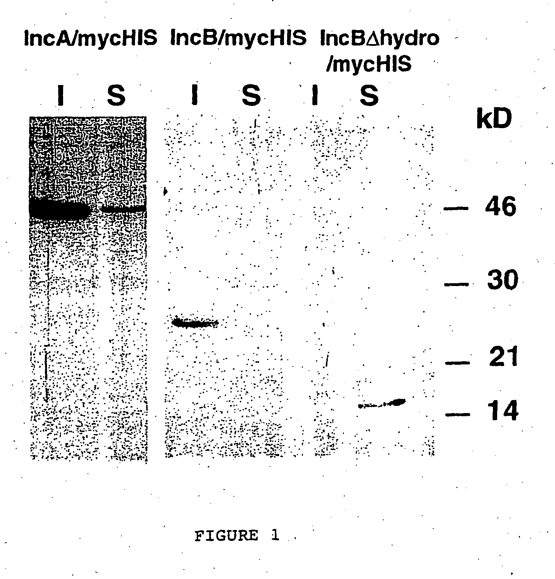

Figure 1: Solubility of chlamydial proteins expressed in *S*. *flexneri*. The recombinant proteins IncA, IncB, and IncB lacking the hydrophobic sequence IncB hydro, carrying a myc/HIS-tag in their C-terminus, were expressed in the wild-type M90T strain. Their solubility was assayed as described in the Materials and Methods section. Equal quantities of the insoluble (I) and soluble (S) fractions were ran on SDS-PAGE and analyzed by Western blot using anti-myc antibodies. One experiment representative of two is shown.

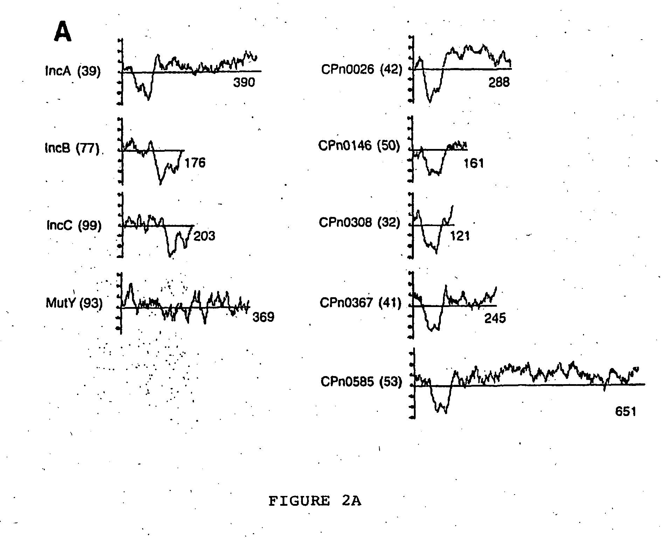

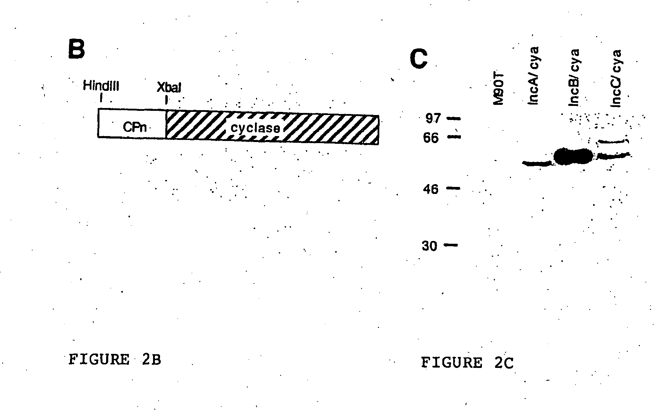

Figure 2: Constructions used in this study. A. hydropathic plot of *C*. *pneumoniae* proteins used to construct the chimeras. The profiles were determined using the algorithm developed by Kyte and Doolittle (Kyte, J., and R.F. Doolittle. (1982). A simple method for displaying the hydropathic character of a protein. J. Mol. Biol. 157: 105-32) with a window size of 22 amino acids. The horizontal axis represents the sequence of the protein and its total number of amino acids is indicated; the vertical axis displays relative hydrophilicity with negative scores indicating hydrophobicity. The number in brackets is the number of amino acids of chlamydial origin in the corresponding Cpn/cya chimera. B. Map of Cpn/cya constructs. The 5' coding region of various *C*. *pneumoniae* proteins was cloned into *Hind*III/*Xba*I sites in frame with the cyclase gene. C. Expression of chimeric proteins in *S*. *flexneri*. Total lysate of wild-type (M90T) or transformed *S*. *flexneri* were analyzed by SDS-PAGE and Western blot using anti-cyclase antibody. The lysate of bacteria expressing IncB/cya was diluted 20-fold as compared to other samples, since the level of expression of this chimera was much higher than that of other chimeras.

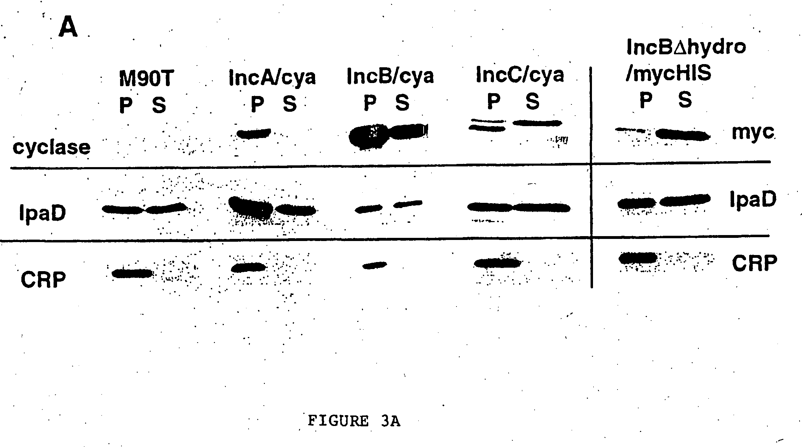

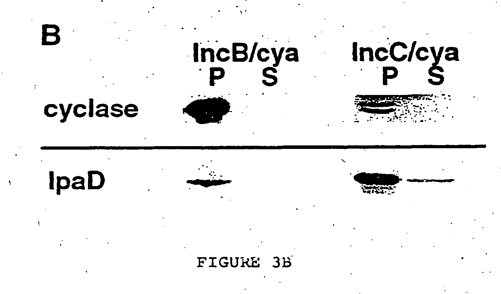

Figure 3: Distribution of Inc/cya chimeras after subcellular fractionation. Exponential cultures of wild-type (A) or of derivatives of wild-type (A) or *mxiD* (B) strains expressing the indicated chimeras were fractionated and the supernatant (S) and pellet (P) fractions were run on SDS-PAGE as described in the Materials and Methods section. The supernatant fraction was concentrated 25-fold as compared to the pellet fraction. After transfer of the proteins, the membrane was cut into three parts, and the presence of the chimera (top); IpaD (middle) and CRP (bottom) in each fraction was probed by Western blot using specific antibodies to each of these proteins. For each lane, the same membrane, analyzed with these three different antibodies, is shown. One experiment representative of three is shown.

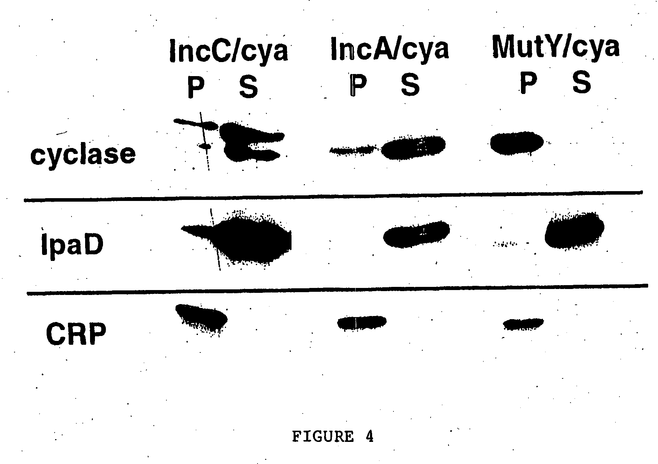

Figure 4: Distribution of IncC/cya, IncA/cya and MutY/cya in *ipaB* derivatives after subcellular fractionation. The samples were prepared and analyzed as described in Figure 3.

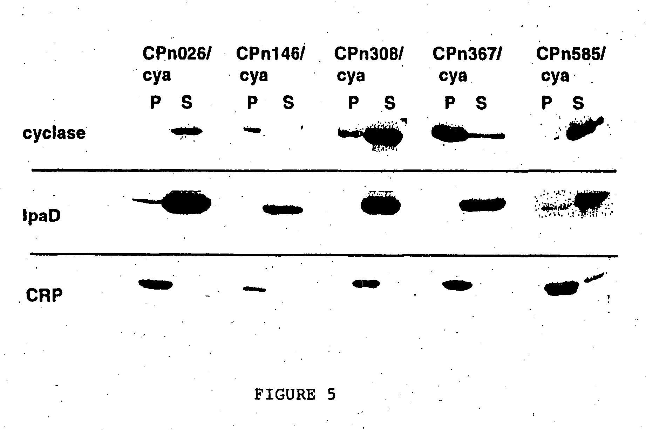

Figure 5: Distribution of several Inc/cya chimeras in transformed *ipaB* derivatives, after subcellular fractionation. The samples were prepared and analyzed as described in Figure 3.

The disclosue of the present invention includes a purified secreted *Chlamydia* polypeptide which is identified by its expression and secretion by the type III secretion pathway of a Gram-negative bacterial strain. The purified polypeptide is selected by a method comprising (a) providing a recombinant expression vector containing at least the DNA coding for the polypeptide of interest; (b) transforming a Gram-negative strain containing a type III secretion pathway with said recombinant vector; (c) expressing this vector in the Gram-negative strain transformed in (b); and (d) detecting the secretion of said DNA expression product; wherein the secretion of said expression product indicates that it corresponds to a secreted *Chlamydia* polypeptide. The detection of the secretion of expression product is made by detecting the presence of said product outside the bacteria.

Alternatively, the purified polypeptide is selected by a method comprising (a) providing a recombinant expression vector comprising at least the DNA coding for the polypeptide of interest fused to a reporter gene; (b) transforming a Gram-negative strain containing a type III secretion pathway with said recombinant vector; (c) expressing this vector in the Gram-negative strain transformed in (b); and (d) detecting the secretion of said reporter gene expression product; wherein the secretion of said expression product indicates that the fused DNA contains at least a polynucleotide corresponding to a secreted *Chlamydia* polypeptide. The detection of the secretion of expression product is made by detecting the presence of said product outside the bacteria.

An object of the present invention is to provide methods of identifying a secreted *Chlamydia* polypeptide. The secreted *Chlamydia* polypeptide is identified by a method comprising (a) providing a recombinant expression vector containing at least DNA coding for the polypeptide of interest; (b) transforming a Gram-negative strain containing a type III secretion pathway with said recombinant vector; (c) expressing said vector in said Gram-negative transformed strain; and (d) detecting the secretion of said DNA expression product; wherein the secretion of said expression product indicates that it corresponds to a secreted *Chlamydia* polypeptide. The detection of the secretion of expression product is made by detecting the presence of said product outside the bacteria.

Alternatively, the secreted *Chlamydia* polypeptide is identified by a method comprising (a) providing a recombinant expression vector containing at least DNA coding for the polypeptide of interest fused to a reporter gene; (b) transforming a Gram-negative strain containing a type III secretion pathway with said recombinant vector; (c) expressing this vector in said transformed Gram-negative strain; and (d) detecting the secretion of said reporter gene expression product; wherein the secretion of said expression product indicates that the fused DNA contains at least a polynucleotide corresponding to a secreted *Chlamydia* polypeptide. The detection of the secretion of expression product is made by detecting the presence of said product outside the bacteria.

Another object of the present invention is to provide methods for screening an active molecule inhibiting the secretion of a secreted *Chlamydia* polypeptide. A method for screening an active molecule inhibiting the secretion of a secreted *Chlamydia* polypeptide comprises (a) providing a recombinant expression vector containing at least DNA coding for the polypeptide the secretion of which is to be inhibited; (b) transforming a Gram-negative strain containing a type III secretion pathway with said recombinant vector; (c) expressing said DNA of said vector in said transformed Gram-negative strain in the presence of the tested molecule; (d) expressing said DNA of said vector in said transformed Gram-negative strain in the absence of the tested molecule; and (e) comparing secretion of the DNA expression product of step (c) and step (d); wherein a decrease of said secretion is indicative of the ability of said tested molecule to inhibit secretion of said secreted *Chlamydia* polypeptide. The detection of the secretion of expression product is made by detecting the presence of said product outside the bacteria.

Alternatively, a method for screening an active molecule inhibiting the secretion of a secreted *Chlamydia* polypeptide comprises (a) providing a recombinant expression vector containing at least DNA coding for the polypeptide the secretion of which is to be inhibited fused to a reporter gene; (b) transforming a Gram-negative strain containing a type III secretion pathway with said recombinant vector; (c) expressing said vector in said transformed Gram-negative strain in the presence of the tested molecule; (d) expressing said vector in said transformed Gram-negative strain in the absence of the tested molecule; and (e) comparing secretion of the expression product of said reporter gene in step (c) and step (d); wherein a decrease of said secretion is indicative of the ability of said tested molecule to inhibit secretion of said secreted *Chlamydia* polypeptide. The detection of the secretion of expression product is made by detecting the presence of said product outside the bacteria.

The disclosure of the present invention includes an immunogenic composition comprising a secreted *Chlamydia* polypeptide, or an immunogenic fragment thereof.

Also provided is a vaccinating composition against *Chlamydia* infection comprising a secreted *Chlamydia* polypeptide, or an immunogenic fragment thereof. For example, said infection is a sexually transmitted disease or contributes to atherosclerosis.

Also provided is a therapeutic composition active against *Chlamydia* infection comprising a molecule which inhibits the secretion of a secreted *Chlamydia* polypeptide.

The disclosure of the present invention includes the provision of an antibody against *Chlamydia* wherein the antibody is directed against a secreted *Chlamydia* polypeptide, or an antigenic fragment thereof.

Another object of the present invention is to provide methods for diagnosing a *Chlamydia* infection in a patient. A method for diagnosing a *Chlamydia* infection in a patient, comprises (a) providing a polypeptide as defined is Claim 1, or an immunogenic fragment thereof, optionally labeled; (b) bringing said polypeptide or immunogenic fragment thereof into contact with a serum sample of said patient; and (c) detecting complexes formed between said polypeptide or immunogenic fragment thereof and antibodies contained in the serum sample; wherein said complexes are indicative of a *Chlamydia* infection in said patient.

Alternatively, a method for diagnosing a *Chlamydia* infection in a patient comprises (a) providing a patient sample of a tissue suspected to be infected by *Chlamydia;* (b) bringing said sample into contact with an antibody against *Chlamydia* wherein said antibody is directed against the purified secreted polypeptide of *Chlamydia* which is identified by its expression and secretion in a Gram-negative strain containing a type III secretion pathway; and (c) detecting antigen-antibody complexion; wherein said complexion is indicative of a *Chlamydia* infection in said patient.

Also provided in the present disclosure is a recombinant plasmid for the expression of a secreted *Chlamydia* polypeptide.

Also provided in the present disclosure is a recombinant Gram-negative bacterial strain transformed by a vector comprising at least a DNA encoding a secreted *Chlamydia* polypeptide.

The disclosure of the present invention includes the provision of a method of preventing or treating a *Chlamydia* infection in a mammal, preferably a human, which comprises administering an effective amount of a purified secreted polypeptide of *Chlamydia* which is identified by its expression and secretion in a Gram-negative strain containing a type III secretion pathway to a mammal in need thereof.

A "polypeptide" as used herein is understood to mean a sequence of several amino acid residues linked by peptide bonds. Such amino acids are known in the art and encompass the unmodified and modified amino acids. In addition, the polypeptide may be modified by one of modifications known in the art such as glycosylation, phosphorylation, etc.

The term "secreted *Chlamydia* polypeptide" as used herein is understood to mean *a Chlamydia* protein, or fragment of the protein, comprising at least 30 amino acids, detectable outside the bacteria.

The term "outside the bacteria" as used herein is understood to mean that a certain portion of the polypeptide of the invention produced by the bacteria has crossed the inner membrane, the periplasm and the outer membrane of the bacteria and is either still bound to the outer membrane, found in the extracellular medium or found inside the host cell.

As used herein, the term "active molecule inhibiting the secretion of a secreted *Chlamydia* polypeptide" is understood to mean a molecule able to reduce, including to totally remove, said secretion by any means. For example, said reduction, including total removal, of secretion may result from an action of said molecule on the type III secretion system or on the expression of said peptide by the bacteria.

The term "type III secretion pathway" as used herein is understood to mean a complex association of various molecules which are necessary for secretion of a polypeptide in a host in which it is normally expressed, as described in Hueck (1998).

The term "immunogenic fragment" as it relates to polypeptides is understood to mean a polypeptide fragment of at least 10 amino acids sufficient to induce an immune response when it is administered to a host eucaryotic organism.

The term "heterologous" as it relates to protein or polypeptide secretion systems is understood to mean that the protein or polypeptide is not normally expressed in a host.

Nucleic acids can be detected utilizing a nucleic acid amplification technique, such as polymerase chain reaction (PCR). Alternatively, the nucleic acid is detected utilizing direct hybridization or by utilizing a restriction fragment length polymorphism.

Hybridization protocols are known in the art and are disclosed, for example, in Sambrook et al., Molecular Cloning: A Laboratory Manual, Cold Spring Harbor Laboratory, New York (1989).

As used herein stringent hybridization conditions are those conditions which allow hybridization between polynucleotides that are 75%, 80%, 85%, 90%, 95%, or 98% as determined using conventional homology programs, an example of which is UWGCG sequence analysis program available from the University of Wisconsin. (Devereaux et al., Nucl. Acids Res. 12: 387-397 (1984)). Such stringent hybridization conditions typically include washing the hybridization in 2X SSC and 0.5% SDS at 65°C (Sambrook et al., Molecular Cloning: A Laboratory Manual, Cold Spring Harbor Laboratory, New York (1989)). Of course, one of skill in the art will recognize that conditions can be varied depending on the length of the polynucleotides to be hybridized and the GC content of the polynucleotides see, for example, Sambrook et al., Molecular Cloning: A Laboratory Manual, Cold Spring Harbor Laboratory, New York (1989).

In this invention "primer" means a polynucleotide which is produced synthetically or biologically and includes a specific nucleotide sequence which permits hybridization to a section containing the target nucleotide sequence.

Defined primers/polynucleotides may be produced by any of several well known methods, including automated solid-phase chemical synthesis using cyanoethyl-phosphoramidite precursors. Other well-known methods for construction of synthetic primers/oligonucleotides may, of curse, be employed. J. Sambrook, E. F. Fritsch and T. Maniatis, Molecular Cloning 11 (2d ed. 1989).

The primers used to amplify the sample nucleic acids may be coupled to a detectable moiety. A preferred example of such a detectable moiety is fluorescein, which is a standard label used in nucleic acid sequencing systems using laser light as a detection system. Other detectable labels can also be employed, however, including other fluorophores, radio labels, chemical couplers such as biotin which can be detected with streptavidin-linked enzymes, and epitope tags such as digoxigenin detected using antibodies. The primers may be modified whereby another nucleotide is added to or substituted for at least one nucleotide in the oligonucleotide. Introduction of known labels such as radioactive substances, enzymes, fluorescence substances, etc. after synthesis of oligonucleotide is also included therein.

Examples of suitable primers for use in the present invention are shown in Table 1.

The inventors have constructed chimeras between the N-terminal domain of several chlamydial proteins and a reporter protein, the calmodulin-dependent adenylate cyclase (Cya) from *B*. *pertussis*, and have tested whether these chimeras were secreted by *S*. *flexneri*. Proteins analyzed in this study can be classified into three categories. (i) IncB and IncC; IncB/cya and IncC/cya chimeras were secreted by derivatives of the wild-type strain but not by derivatives of the secretion deficient *mxiD* mutant, demonstrating that these two proteins were secreted by a type III mechanism. Moreover, IncC/cya was more efficiently secreted by the *ipaB* mutant, than by the wild-type strain, confirming that this chimera was secreted by the type III secretion machinery. In contrast, similar amounts of IncB/cya were secreted by the wild-type and *ipaB* strains. It is noteworthy that IncB/cya was expressed at a much higher level than any other Inc/cya chimera (see Fig. 2C) and bacteria expressing IncB/cya grew more slowly than those expressing the other chimeras. Overproduction of IncB/cya might have led to the formation of aggregates that were not competent for secretion. (ii) IncA, Cpn0026, Cpn0308, and Cpn0585; although the corresponding chimeras were not detected in the supernatant of derivatives of the wild-type strain, they were present in the supernatant of derivatives of the *ipaB* mutant. Their secretion was specific because two cytosolic markers, CRP and MutY/cya, were not detected in the supernatant of these strains. These results indicated that, although the corresponding chimeras were not as efficiently secreted as IncB/cya and IncC/cya chimeras, these four proteins were nevertheless secreted by a type III mechanism. (iii) Cpn0146 and Cpn0367; the corresponding chimeras were not detected in significant amounts in the culture medium of derivatives of either the wild-type or the *ipaB* strains. This might indicate that Cpn0146 and Cpn0367 are not substrates of the type III secretion machinery of *C. pneumoniae*. Alternatively, these proteins might be secreted by the type III secretion pathway of *C. pneumoniae* but their secretion signal was either not present, or not accessible, in the chimeras constructed here or was not recognized by the type III secretion machinery of *S. flexneri*.

IncB and IncC, both of which were secreted by derivatives of the wild-type *S. flexneri* strain, have one feature that distinguishes them from other Inc proteins and other proteins examined in the study: their hydrophobic domain is located at their C-terminal end, whereas the hydrophobic domain of the other proteins is located only 30 to 50 residues downstream from their N-terminus (Fig. 1). These two topologies might reflect the existence of two subfamilies of Inc proteins, with possibly two different types of secretion signal. The secretion signal of the first family (including IncB, IncC and potentially a few other proteins) might be entirely located upstream from the hydrophobic domain whereas the secretion signal of the second family (including IncA, Cpn0026, Cpn0308, Cpn0585, and most other Inc proteins) might be composite, part of it being present upstream from the hydrophobic domain and the other part being present downstream from that domain. This putative second signal which was not present in the construct, might not be necessary for recognition by the type III secretion machinery but important for efficient secretion.

Analysis of the proteins encoded by the *C. pneumoniae* genome revealed the presence of over 50 proteins that have a unique hydrophobic domain longer than 40 residues (Bannantine *et al*., 2000) and the inventors' own results). In this study, the inventors have shown that three out of five such proteins carry a secretion signal that is recognized by the type III secretion of *S. flexneri* and are, therefore, candidates for being inserted into the membrane of the inclusion. This suggests that orphan proteins that have a long hydrophobic domain have a high probability to be secreted by the type III secretion machinery of *Chlamydia* spp. and to belong to the Inc family. This is in agreement with a recent report in which several putative Inc proteins of *C. trachomatis*, chosen on the basis of their hydropathic profile, were found associated with the inclusion membrane (Bannantine *et al*., 2000).

The hydrophobic domain of Inc proteins is likely to allow these proteins to insert into the membrane of the inclusion. When expressed in *S. flexneri*, full length forms of IncA and IncB were not soluble, while a truncated form of IncB, in which the hydrophobic domain was deleted, was soluble. In *Chlamydia,* chaperone molecules might be involved in the solubilization and subsequent secretion of Inc proteins. Another possibility is that translation may be tightly coupled to secretion of the Inc proteins and that these proteins do not accumulate in the bacterial cytosol. A better understanding of the mechanism of type III secretion is needed to address these questions in the case of *Chlamydia*. Moreover, the length of the hydrophobic domain of Inc proteins, which is more than twice the number of amino acid residues that are required to span a biological membrane, is unusual and the topology of their association with the inclusion is not known. Finally, whether other chlamydial proteins that do not belong to the Inc family are also inserted into the membrane of the inclusion or translocated into the cytosol remains to be investigated.

Genes coding for type III secretion apparatuses of *C. trachomatis and C. pneumoniae* are found at several locations on the genomes, unlike those of other pathogens in which they are clustered (Hsia *et al*., 1997; Hueck, 1998; Stephens, *et al*., 1998; Subtil, *et al*., 2000). If they have been acquired in one cluster by horizontal transfer, as it is the case for other pathogens, their present dispersal on the genome suggests that type III secretion is a very old acquisition for *Chlamydia* spp. It is therefore remarkable that chlamydial secretion signals can be recognized by the secretion machinery of other pathogens such as *S. flexneri*. In the absence of genetic tools to manipulate the members of *Chlamydia* spp., this finding opens new approaches for the study of chlamydial genes.

The polypeptides provided are administered in a dose which is effective to vaccinate a mammal, preferably a human, against chlamydial infection or to treat a mammal, preferably a human, having a chlamydial infection. As used herein, an effective amount of the polypeptides to achieve this goal is generally from about 2 ng to 2 mg/kg of body weight per week, preferably about 2 µg/kg per week. This range includes all specific values and subranges there between.

The polypeptides identified by the disclosed method may be administered as a pharmaceutical composition containing the polypeptide compound and a pharmaceutically acceptable carrier or diluent. The active materials can also be mixed with other active materials which do not impair the desired action and/or supplement the desired action. The active materials according to the present invention can be administered by any route, for example, orally, parenterally, intravenously, intradermally, subcutaneously, or topically, in liquid or solid form.

For the purposes of parenteral therapeutic administration, the active ingredient may be incorporated into a solution or suspension. The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparation can be enclosed in ampoules, disposable syringes or multiple dose vials made of glass or plastic.

Another mode of administration of the polypeptides identified by the disclosed method is oral. Oral compositions will generally include an inert diluent or an edible carrier. For the purpose of oral therapeutic administration, the aforesaid polypeptides may be incorporated with excipients and used in the form of tablets, gelatine capsules, troches, capsules, elixirs, suspensions, syrups, wafers, chewing gums.and the like. Compositions may be prepared according to any method known to the art for the manufacture of pharmaceutical compositions and such compositions may contain one or more agents selected from the group consisting of sweetening agents, flavoring agents, coloring agents and preserving agents. Tablets containing the active ingredient in admixture with nontoxic pharmaceutically acceptable excipients which are suitable for manufacture of tablets are acceptable. These excipients may be, for example, inert diluents, such as calcium carbonate, sodium carbonate, lactose, calcium phosphate or sodium phosphate granulating and disintegrating agents, such as maize starch, or alginic acid; binding agents, such as starch, gelatin or acacia; and lubricating agents, such as magnesium stearate, stearic acid or talc. Tablets may be uncoated or may be coated by known techniques to delay disintegration and adsorption in the gastrointestinal tract and thereby provide a sustained action over a longer period. For example, a time delay material such as glyceryl monostearate or glyceryl distearate alone or with a wax may be employed. Formulations for oral use may also be presented as hard gelatin capsules wherein the active ingredient is mixed with an inert solid diluent, for example calcium carbonate, calcium phosphate or kaolin, or as soft gelatin capsules wherein the active ingredient is mixed with water or an oil medium, such as peanut oil, liquid paraffin or olive oil.

The tablets, pills, capsules, troches and the like may contain the following ingredients: a binder such as microcrystalline cellulose, gum tragacanth or gelatin; an excipient such as starch or lactose, a disintegrating agent such as alginic acid, Primogel, corn starch and the like; a lubricant such as magnesium stearate or Sterotes; a glidant such as colloidal silicon dioxide; and a sweetening agent such as sucrose or saccharin or flavoring agent such as peppermint, methyl salicylate, or orange flavoring may be added. When the dosage unit form is a capsule, it may contain, in addition to material of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active polypeptides, sucrose as a sweetening agent and certain preservatives, dyes and colorings and flavors. Materials used in preparing these various compositions should be pharmaceutically or veterinarially pure and non-toxic in the amounts used.

Aqueous suspensions of the disclosure contain the active materials in admixture with excipients suitable for the manufacture of aqueous suspensions. Such excipients include a suspending agent, such as sodium carboxymethylcellulose, methylcellulose, hydroxypropylethyl cellulose, sodium alginate, polyvinylpyrrolidone, gum tragacanth and gum acacia, and dispersing or wetting agents such as a naturally occurring phosphatide (e.g., lecithin), a condensation product of an alkylene oxide with a fatty acid (e.g., polyoxyethylene stearate), a condensation product of ethylene oxide with a long chain aliphatic alcohol (e.g., heptadecaethylene oxycetanol), a condensation product of ethylene oxide with a partial ester derived from a fatty acid and a hexitol (e.g., polyoxyethylene sorbitol mono-oleate), or a condensation product of ethylene oxide with a partial ester derived from fatty acid and a hexitol anhydride (e.g., polyoxyethylene sorbitan mono-oleate). The aqueous suspension may also contain one or more preservatives such as ethyl or n-propyl p-hydroxybenzoate, one or more coloring agents, one or more flavoring agents and one or more sweetening agents, such as sucrose, aspartame, saccharin, or sucralose.

Oil suspensions may be formulated by suspending the active ingredient in a vegetable oil, such as arachis oil, olive oil, sesame oil or coconut oil, or in a mineral oil such as liquid paraffin. The oil suspensions may contain a thickening agent, such as beeswax, hard paraffin or cetyl alcohol. Sweetening agents may be added to provide a palatable oral preparation. These compositions may be preserved by the addition of an antioxidant such as ascorbic acid.

Dispersible powders and granules of the disclosure suitable for preparation of an aqueous suspension by the addition of water may be formulated from the active ingredients in admixture with a dispersing, suspending and/or wetting agent, and one or more preservatives. Suitable dispersing or wetting agents and suspending agents are exemplified by those disclosed above. Additional excipients, for example sweetening, flavoring and coloring agents, may also be present.

The compositions of the disclosure may also be in the form of oil-in-water emulsions. The oily phase may be a vegetable oil, such as olive oil or arachis oil, a mineral oil, such as liquid paraffin, or a mixture of these. Suitable emulsifying agents include naturally occurring gums, such as gum acacia and gum tragacanth, naturally occurring phosphatides, such as soybean lecithin, esters or partial esters derived from fatty acids and hexitol anhydrides, such as sorbitan mono-oleate, and condensation products of these partial esters with ethylene oxide, such as polyoxyethylene sorbitan mono-oleate. The emulsion may also contain sweetening and flavoring agents.

Syrups and elixirs may be formulated with sweetening agents, such as glycerol, sorbitol or sucrose. Such formulations may also contain a demulcent, a preservative, a flavoring or a coloring agent.

The compositions of the disclosure may be in the form of a sterile injectable preparation, such as a sterile injectable aqueous or oleaginous suspension. This suspension may be formulated according to the known art using those suitable dispersing or wetting agents and suspending agents which have been mentioned above. The sterile injectable preparation may also be a sterile injectable solution or suspension in a nontoxic parenterally acceptable diluent or solvent, such as a solution of 1,3-butanediol. Among the acceptable vehicles and solvents that may be employed are water and Ringer's solution, an isotonic sodium chloride. In addition, sterile fixed oils may conventionally be employed as a solvent or suspending medium. For this purpose any bland fixed oil may be employed including synthetic mono- or diglycerides. In addition, fatty acids such as oleic acid may likewise be used in the preparation of injectables. Sterilization may be performed by conventional methods known to those of ordinary skill in the art such as by aseptic filtration, irradiation or terminal sterilization (e.g. autoclaving).

Aqueous formulations (i.e oil-in-water emulsions, syrups, elixers and injectable preparations) may be formulated to achieve the pH of optimum stability. The determination of the optimum pH may be performed by conventional methods known to those of ordinary skill in the art. Suitable buffers may also be used to maintain the pH of the formulation.

The purified polypeptides may also be administered in the form of suppositories for rectal administration of the drug. These compositions can be prepared by mixing the drug with a suitable nonirritating excipient which is solid at ordinary temperatures but liquid at the rectal temperatures and will therefore melt in the rectum to release the drug. Non-limiting examples of such materials are cocoa butter and polyethylene glycols.

An *ipaB* mutant strain of *S. flexneri* expresssing IncA 1 ipaB was deposited at C.N.C.M., Institut Pasteur, 25, Rue de Docteur Roux, F-75724, Paris Cedex 15, France, on December 13, 2000, with accession number I-2592.

A bacterial strain containing the vector pUC19cya was deposited at C.N.C.M., Institut Pasteur, 25, Rue de Docteur Roux, F-75724, Paris Cedex 15, France, on December 13, 2000, with accession number 1-2593.

An ipaB⁻ mutant strain of *S. flexneri* designated SF620 was deposited at C.N.C.M., Institut Pasteur, 25, Rue de Docteur Roux, F-75724, Paris Cedex 15, France, on December 13, 2000, with accession number I-2594.

### EXAMPLES

Having generally described this invention, a further understanding can be obtained by reference to certain specific examples which are provided herein for purposes of illustration only, and are not intended to be limiting unless otherwise specified.

### Example 1

### Materials and Methods

### Bacterial strains and reagents

Strain M90T is the virulent, wild-type strain of *S. flexneri* 5 (Sansonetti *et al*., 1982). Strains SF401 and SF620 are derivatives of M90T in which the *mxiD and ipaB* genes, respectively, have been inactivated (Allaoui, A., P.J. Sansonetti, and C. Parsot. (1993). MxiD, an outer membrane protein necessary for the secretion of the Shigella flexneri lpa invasins. Mol. Microbiol. 7: 59-68; Ménard, R., P. Sansonetti and C. Parsot. (1994). The secretion of the Shigella flexneri Ipa invasins is activated by epithelial cells and controlled by IpaB and IpaD. EMBO J. 13: 5293-302). The *E. coli* strain TG1 (Sambrook et al., Molecular Cloning: A Laboratory Manual, Cold Spring Harbor, New York, 1989) was used for plasmid constructions. *S. flexneri and E. coli* strains were grown in Luria-Bertani (LB). Ampicillin was used at 0.1 mg/ml. Monoclonal antibodies against the calmodulin-dependent adenylate cyclase (Brézin *et al*, 1987), the myc tag 9E10F5 (Calbiochem, Germany) and polyclonal antibodies 280III against the cAMP receptor protein (CRP) (Biville, F. and Guiso, N. (1985). Evidence for the presence of cAMP, cAMP receptor and transcription termination factor Rho in different Gram-negative bacteria (J. Gen. Microbiol. 131(11): 2953-2960) were used. Anti-IpaD antibody was used as described (Ménard, R., P.J. Sansonetti, and C. Parsot. (1993). Nonpolar mutagenesis of the ipa genes defines IpaB, IpaC, and IpaD as effectors of Shigella flexneri entry into epithelial cells. J. Bacteriol. 175: 5899-906). Horseradish peroxidase-linked secondary antibodies for enhanced chemiluminescence were obtained from Amersham Pharmacia Biotech (Orsay, France). Alkaline phosphatase-linked secondary antibodies for enhanced chemifluorescence were obtained from Pierce (Rockford, IL, USA).

### Production of antibodies

Methods for the production of antibodies directed to a specific peptide or fragment thereof are well known by those of skill in the art. Antibodies can be obtained by injecting an animal with an immunogenic peptide of the invention, or an immunogenic fragment thereof, and recovering the antibodies which are able to complex with said immunogenic peptide or fragment thereof from said animal. Examples of such methods are disclosed in Antibodies, A Laboratory Manual, Harlow and Lane, Cold Spring Harbor Press, 1988, herein incorporated by reference.

### Construction of recombinant plasmids expressing hybrid proteins

Genomic DNA from *C. pneumoniae* strain TW183 (American Type Culture Collection, Virginia, U.S.A.; ATCC No. VR-2282) was prepared from purified bacteria using the RapidPrep Micro Genomic DNA isolation kit (Amersham Pharmacia Biotech). The 5' part of different chlamydial genes, including about 30 nucleotides located upstream from the proposed translation start sites and the first 30 to 99 codons, were amplified by PCR using the primers listed in Table 1. For the myc/HIS tagged constructs, the forward and reverse primers contained additional *Xho*I and *EcoR*I sites, respectively, to allow cloning of the PCR fragments between the *Xho*I *and Eco*RI sites of the pTrcHis2A vector (Invitrogen, Groningen, The Netherlands). For the Inc/cya constructs, the forward and reverse primers contained additional HindIII *and Xba*I sites, respectively, to allow cloning of the PCR fragments between the HindIII *and Xba*I sites of the puc19cya vector. This vector was constructed by cloning the *Xba*I-*EcoR*I fragment of plasmid pMS 109, which carried the *cya* gene of *Bordetella pertussis* (Sory, M.P., and G.R. Cornelis. (1994). Translocation of a hybrid YopE-adenylate cyclase from Yersinia enterocolitica into HeLa cells. Mol. Microbiol. 14: 583-94), between *Xba*I *and Eco*RI sites of the pUC 19 vector. In the recombinant plasmids, transcription of the hybrid genes was under the control of the *lac* promoter of the vector. Recombinant plasmids were amplified in *E. coli* TG1 and the sequence of all constructs was checked by sequencing. Methods of sequencing nucleic acids are known in the art and are described in, for example, Sambrook et al., Molecular Cloning: A Laboratory Manual, Cold Spring Harbor Laboratory, New York (1989) and Current Protocols in Molecular Biology, Ausebel et al, eds., John Wiley and Sons, Inc., New York (2000).

| TABLE 1: Oligonucleotides used to construct the Inc/cya chimeras described in this study. Restriction sites used for cloning are underlined.Chime ra | **forward primer** | **reverse primer** |
|---|---|---|
| **IncA/mycHIS** | TGACCTCGAGTTAACCTATTAAG GATAAAATT (SEQ ID NO:1) | GACTGAATTCGTTGCTCTATCT ACGGGTGA (SEQ ID NO:13) |
| **IncB/mycHIS** | GATCCTCGAGTTAATCTATTTTT AGATAGG (SEQ ID NO:2) | GACTGAATTCCTTGTTGTACGG ACAGTAAT (SEQ ID NO:14) |
| **IncB hydro/mycHIS** | GATCCTCGAGTTAATCTATTTTT AGATAGG (SEQ ID NO:3) | CTAGGAATTCGATTGAACAGTA ACAGATCC (SEQ ID NO:15) |
| **IncA/cya** | GACTAAGCTTGTAACCTATTAAG GATAAAATT (SEQ ID NO:4) | GACTTCTAGAAATGATAACCTT TTCAATGAA (SEQ ID NO:16) |
| **IncB/cya** | GACTAAGCTTGTAATCTATTTTT AGATAGGAA (SEQ ID NO:5) | GACTTCTAGATCCAGGTTTTTC GGAAGCAGA (SEQ ID NO:17) |
| **IncC/cya** | GACTAAGCTTGTAAGTAAAAAA CACAAAAAAT (SEQ ID NO:6) | GACTTCTAGATATTTGAGCTGG TACAACAGG (SEQ ID NO:18) |
| **mutY/cya** | GACTAAGCTTGCATTTGATAATT GCATAAA (SEQ ID NO:7) | GACTTCTAGACGCTCGAGAATA ATAACCC (SEQ ID NO:19) |
| **CP026/cya** | GACTAAGCTTGAATACATAAGCT GTTC (SEQ ID NO:8) | GACTTCTAGAAATGATTAGGTA AGCAATG (SEQ ID NO:20) |
| **CP 146/cya** | GACTAAGCTTAAAGTGTTTGAG ATGAATT (SEQ ID NO:9) | GACTTCTAGACGCTCCCAACCC CAGAGTC (SEQ ID NO:21) |
| **CP308/cya** | GACTAAGCTTATTATATAGACAG ATTAAAAT (SEQ ID NO:10) | GACTTCTAGACTTAAAAAATAC CCAGGAACA (SEQ ID NO:22) |
| **CP367/cya** | GACTAAGCTTACAACAAATTAAG ATATAATC (SEQ ID NO:11) | GACTTCTAGATTTTATTATTTTA GCAATCAC (SEQ ID NO:23) |
| **CP585/cya** | GACTAAGCTTGTAAATTGGAGA TTGTAGTAGC (SEQ ID NO:12) | GACTTCTAGAAACAATTGTATG ATTCCATCC (SEQ ID NO:24) |

### Subcellular fractionation and protein analysis

To analyze the solubility of recombinant proteins expressed in *S. flexneri*, 1 ml of an overnight culture was inoculated in 30 ml of LB and incubated at 37 C for 1 h before inducing the expression of the recombinant protein by adding 0.4 mM isopropyl-D-thiogalactopyranoside (IPTG). Bacteria were harvested 3 h after induction, resuspended in 2 ml 100 mM NaCl, 10 mM Hepes pH 7.2 (sonication buffer) and sonicated. The cell lysate was centrifuged for 5 min at 1,700 x g to eliminate non-lyzed bacteria, and the supernatant was centrifuged for 30 min at 540,000 x g. The pellet was resuspended in 2 ml of sonication buffer and equal volumes of samples of the pellet (insoluble fraction) and supernatant (soluble fraction) were analyzed by electrophoresis in 12 % polyacrylamide gels in the presence of sodium dodecyl sulfate (SDS-PAGE). After electrophoresis, proteins were transferred on a PVDF membrane (Millipore Corporation, Bedford, MA), and the membrane was used for blotting with anti-myc 9E10F5 antibodies. Western blotting and revelation were performed by enhanced chemiluminescence (Amersham Pharmacia Biotech) according to the manufacturer's instructions.

Analysis of secreted proteins was performed as previously described Allaoui, A., P.J. Sansonetti, and C. Parsot. (1993). MxiD, an outer membrane protein necessary for the secretion of the Shigella flexneri lpa invasins. Mol. Microbiol. 7: 59-68). Briefly, 1 ml of an overnight culture was inoculated in 30 ml of LB and incubated at 37 C for 3 h (0.4 mM IPTG was added for expression of the IncB hydro/mycHIS chimera). Bacteria were harvested by centrifugation and the culture supernatant was filtered through 0.22 µm filters. Proteins present in 25 ml of the filtrates were precipitated by the addition of 1/10 (v/v) trichloroacetic acid, and resuspended in 0.5 ml of sample buffer for electrophoresis. Equal volumes of samples of the bacterial pellet and culture supernatants, the latter being concentrated 25-fold as compared to the pellet, were analyzed by SDS-PAGE. After electrophoresis, proteins were transferred on a PVDF membrane, and the membrane was used for blotting with anti-cyclase or anti-myc, anti-IpaD and anti-CRP antibodies respectively.

*C. trachomatis* and *C. pneumoniae* genomes can be found on the websites: http://chlamydia.www.berkeley.edu:4231 and http://www.tigr.org/tdb/.

### Results

### Expression of tagged chlamydial proteins in S. flexneri

C. *pneumoniae* proteins Cpn0186 and Cpn0291 show some sequence similarity with the IncA and IncB proteins respectively, of *C. trachomatis and C. psittaci*. The inventors constructed tagged forms of these proteins by adding a short myc/HIS epitope at their C-terminus. When expressed in wild-type *S. flexneri*, both proteins were found to be insoluble as determined by subcellular fractionation, SDS-PAGE and immunoblotting using anti-myc antibody (Fig. 1). In contrast, IncB hydro/mycHIS, in which the hydrophobic domain of IncB was deleted, was present in the soluble fraction, showing that the insolubility of the full length protein is due to the presence of the hydrophobic domain. Because of their large hydrophobic domain, most Inc proteins are likely to be insoluble when expressed in *S. flexneri*, which would prevent the study of their secretion. To circumvent this difficulty, the inventors decided to express chimeric proteins between Inc proteins and a reporter molecule, which did not include the hydrophobic domain of Inc proteins.

### Construction of chimeras between chlamydial proteins and the cyclase reporter

The inventors hypothesized that the signal for secretion of proteins by the putative chlamydial type III secretion machinery might be located either within the first codons of the mRNA or within the first residues of the secreted proteins and that this secretion signal might be recognized by a heterologous secretion machinery, such as that of *S. flexneri*. To test these hypotheses, the inventors constructed hybrid genes by fusing the 5' part of the chlamydial gene of interest in frame with the *cya* gene of *B. pertussis* encoding the calmodulin-dependent adenylate cyclase (Cya). This reporter protein was chosen as it had already been used as a reporter system for type III secretion in *Yersinia* and *Salmonella* spp. (Jones, M.A., M.W. Wood, P.B. Mullan, P.R. Watson, T.S. Wallis, and E.E. Galyov. (1998). Secreted effector proteins of Salmonella dublin act in concert to induce enteritis. Infection & Immunity. 66: 5799-804; Sory, M.P., and G.R. Cornelis. (1994). Translocation of a hybrid YopE-adenylate cyclase from Yersinia enterocolitica into HeLa cells. Mol. Microbiol. 14: 583-94). Plasmids producing hybrid proteins from the constitutive *lac* promoter of the vector and the *Chlamydiae* translation start sites were introduced into *S. flexneri* strains expressing various phenotypes with respect to type III secretion, i.e. in which secretion was either low (wild-type strain), deficient (*mxiD* mutant) or deregulated (*ipaB* mutant) (Allaoui, A., P.J. Sansonetti, and C. Parsot. (1993). MxiD, an outer membrane protein necessary for the secretion of the Shigella flexneri lpa invasins. Mol. Microbiol. 7: 59-68; Ménard, R., P.J. Sansonetti, and C. Parsot. (1993). Nonpolar mutagenesis of the ipa genes defines IpaB, IpaC, and IpaD as effectors of Shigella flexneri entry into epithelial cells. J. Bacteriol. 175: 5899-906). Production and secretion of hybrid proteins was investigated by SDS-PAGE and immunoblotting using an anti-cyclase monoclonal antibody.

To investigate whether *C. pneumoniae* IncA (CPn0186), IncB (CPn0291), and IncC (CPn0292) carried signals that might be recognized by the *S. flexneri* type III secretion machinery, the inventors constructed recombinant plasmids that expressed hybrid proteins consisting in the amino terminal part of IncA, IncB and IncC, located upstream from the hydrophobic domain present in each of these proteins, and the adenylate cyclase domain (Fig. 2A). These recombinant proteins were designated IncA/cya, IncB/cya and IncC/cya, respectively (Fig. 2B). *S. flexneri* wild-type strain M90T was transformed with these constructs and expression of the hybrid proteins was probed by Western blot using anti-cyclase antibody (Fig. 2C). IncA/cya and IncB/cya were expressed as single products with the expected sizes and IncC/cya appeared as a doublet, which might correspond to different translation initiation sites.

### IncB/cya and IncC/cya are secreted by the S. flexneri type III secretion machinery

Although the activity of the *S. flexneri* type III secretion machinery is regulated by contact with host cells, secretion can also be observed using bacteria growing in broth (Allaoui, A., P.J. Sansonetti, and C. Parsot. (1993). MxiD, an outer membrane protein necessary for the secretion of the Shigella flexneri lpa invasins. Mol. Microbiol. 7: 59-68; Ménard, R., P. Sansonetti and C. Parsot. (1994). The secretion of the Shigella flexneri Ipa invasins is activated by epithelial cells and controlled by IpaB and IpaD. EMBO J. 13: 5293-302). Subcellular fractionations were performed on exponentially growing cultures to test whether Inc/cya chimeras were secreted by wild-type *S. flexneri*. Proteins present in the supernatant fraction were concentrated 25-fold relative to those present in the pellet fraction, and equal amounts of each sample were analyzed by SDS-PAGE and Western blotting using specific antibodies (Fig. 3A). As a control, the inventors checked the ability of these transformed strains to secrete IpaD, one of the *S. flexneri* proteins that is secreted by the wild-type strain. Similar amounts of IpaD were present in the culture supernatant of the wild-type and recombinant strains, indicating that production of the Inc/cya proteins had no effect on the activity of the type III secretion machinery. The inventors detected the presence of IncB/cya and IncC/cya in the supernatant fractions using anti-cyclase antibody, indicating that these chimeras were secreted by *S. flexneri*. The presence of these proteins in the supernatant fraction was not due to a contamination by lysed bacteria, since the cyclic AMP receptor protein (CRP), a cytosolic protein, was only found in the pellet fraction. IncA/cya was not detected in the supernatant, indicating that this chimera was either not secreted by wild-type *S. flexneri*, or secreted in amounts too low to be detected.

To verify that the secretion of IncB/cya and IncC/cya was not an artifact of the Inc/cya fusion, the inventors tested the subcellular localization of the IncB hydro/mycHIS chimera mentioned above, in which the Cya domain has been replaced by a myc/HIS tag. When expressed in the wild-type M90T strain, the IncB hydro/mycHIS chimera was detected in the culture supernatant, demonstrating that secretion is not an artifact of the Inc/cya fusion (Fig. 3A).

To test whether secretion of IncB/cya and IncC/cya was dependent on the type III secretion machinery, plasmids expressing these proteins were introduced into a *mxiD* mutant, in which type III secretion is totally impaired (Allaoui, A., P.J. Sansonetti, and C. Parsot. (1993). MxiD, an outer membrane protein necessary for the secretion of the Shigella flexneri Ipa invasins. Mod. Microbiol. 7: 59-68). Neither IncB/cya nor IncC/cya were detected in the culture supernatants of derivatives of the *mxiD* mutant (Fig. 3B) which, as expected, did not secrete IpaD. These experiments demonstrate that IncB/cya and IncC/cya are secreted by a type III machinery in wild-type *S*. *flexneri*.

### IncA also carries a signal for secretion by a type III machinery

The lack of detection of IncA/cya in the culture supernatant of the wild-type strain might be due to a lower secretion efficiency of this protein in the experimental assay as compared to the IncB/cya and IncC/cya hybrids. To investigate this possibility, plasmids expressing IncC/cya and IncA/cya proteins were introduced into a *S*. *flexneri ipaB* mutant, a strain in which the activity of the type III secretion machinery is deregulated. Indeed, only a small proportion of the Ipa proteins that are produced are secreted by the wild-type strain in the absence of external inducers, whereas 100 % of the Ipa proteins are secreted by an *ipaB* mutant (Ménard, R., P. Sansonetti and C. Parsot. (1994). The secretion of the Shigella flexueri Ipa invasins is activated by epithelial cells and controlled by IpaB and IpaD. EMBO J. 13: 5293-302). This mutant had been constructed by replacing most of the *ipaB* gene by a non-polar *aphA-3* cassette (Ménard, R., P.J. Sansonetti, and C. Parsot. (1993). Nonpolar mutagenesis of the ipa genes defines IpaB, IpaC, and IpaD as effectors of Shigella flexneri entry into epithelial cells. J. Bacteriol. 175: 5899-906). Secretion, of IncC/cya was more efficient in the *ipaB* mutant than in the wild-type, confirming that this chimera was secreted by the type III secretion pathway (Fig. 4). Moreover, IncA/cya was also present in the supernatant of the derivative of the *ipaB* mutant. Detection of IncA/cya in the culture medium was not due to contamination by lysed bacteria since CRP was found only in the pellet. As an additional control, the inventors constructed a recombinant plasmid expressing a hybrid protein that consists in the first 93 residues of *C*. *pneumoniae* adenosine glycosylase (MutY) and Cya. MutY is a cytosolic protein involved in DNA repair. When expressed in the *ipaB* mutant, MutY/cya was found only in the pellet (Fig. 4). These results indicate that IncA/cya also carries a secretion signal that is recognized by the type III secretion machinery of *S*. *flexneri*.

### Identification of proteins secreted by Chlamydia based on their hydropathic profile

IncA, IncB, and IncC do not exhibit any sequence similarity; however, each of these proteins has a hydrophobic domain of more than 40 amino acids (Fig. 2). From a global analysis of the *C*. *pneumoniae* genome, the inventors detected more than 50 hypothetical proteins with such a long hydrophobic domain. To test whether these proteins might be targets of the type III secretion machinery of *C*. *pneumoniae*, the inventors constructed chimeras between 5 of these chlamydial proteins, coded by genes which are not clustered on the genome, and the cyclase reporter, i.e. CPn026/cya, CPn146/cya, CPn308/cya, CPn367/cya and CPn585/cya (Fig. 2). These constructs were transferred into the wild-type and *ipaB* strains, and localization of the chimeras was analyzed by Western blot after subcellular fractionation. None of the chimeras were detected in the supernatant of derivatives of the wildtype strain (data not shown). However, CPn026/cya, CPn308/cya, and CPn585/cya were present in the supernatant of derivatives of the *ipaB* mutant in which type III secretion is more efficient (Fig. 5). Quantification of the immunoblots using enhanced chemifluorescence showed that 5% (CPn308/cya) to 50% (CPn585/cya) of the chimera were present in the supernatant fraction of *ipaB* derivatives. These proportions are lower than for IpaD, for which 100% is secreted in this strain. This may explain why the chimeras were not detected in the supernatant of wild type *S*. *flexneri*, in which only about 5% of IpaD is secreted (Ménard, R., P. Sansonetti and C. Parsot. (1994). The secretion of the Shigella flexneri Ipa invasins is activated by epithelial cells and controlled by IpaB and IpaD. EMBO J. 13: 5293-302). The two other chimeras were not detected in the supernatant (CPn146/cya) or at a level too low to be significant (CPn367/cya, less than 1% detected in the supernatant fraction). Accordingly, three orphan proteins, out of five proteins selected from the *C*. *pneumoniae* database on the basis of their hydropathic profile, were secreted by the type III secretion machinery of *S. flexneri*.

### Example 2

Detecting secreted proteins by the type III secretion system by *Shigella flexneri* colonies. The secretion of 122 proteins coded by *C*. *pneumoniae* strain CWL029 genome were tested (Nature Genetics (1999) 21:385, the gene sequences can be found at http://chlamydia-www.berkeley.edu:4231/ and in GenBank under the accession number AE 001363, which are incorporated herein by reference). Chimeras consisted of the fusion between the amino terminal domain of chlamydial proteins and the cyclase reporter gene (CPn/cya chimera) as described in Subtil et al 2001. Forty-three chimeras were found to be secreted. They can be classified into 4 categories:
Category 1 -Chimeras in which the amino-terminal part is provided by chlamydial proteins containing a bilobed hydrophobic domain and are therefore putative members of the family of Inc proteins (Subtil et al (2001) Mol. Microbiol. 39:792-800).
   Secreted proteins of this category are:
      CPn0026, CPn0067, CPn0130, CPn0146, CPn0174, CPn0186, CPn0211, CPn0243, CPn0277; CPn0284, CPn0292, CPn0357, CPn0365, Cpn1027
Category 2 - Chimeras in which the amino-terminal part is provided by a chlamydial protein containing an hydrophobic domain that is not so well defined but may be used for insertion into the inclusion membrane and may therefore also be part of the family of Inc proteins. Secreted proteins of this category are:
   CPn0028, CPn0049, CPn0066, CPn0132, CPn0220, CPn0223, CPn0226, CPn0267, CPn0648, Cpn0829
Category 3 -Chimeras which do not belong to the Inc family of proteins and in which the amino-terminal part is coded by a gene specific to *C*. *pneumoniae*. Secreted proteins of this category are:
   CPn0009, CPn0012, CPn0063, CPn0167, CPn0175, CPn0181.
Category 4 - Chimeras which do not belong to the Inc family of proteins and in which the amino-terminal part is coded by a gene that has an homolog in *C*. *trachomatis* and/or *C*. *psittaci* genomes (*C*. *trachomatis* serovar D gene sequences can be found at http://chlamydia-www.berkeley, edu:4231/ Science (1998) 282:754, and in GenBank sunder the accession number AE 001273, which are incorporated herein by reference; *C*. *psittaci* GPIC strain unfinished genome was provided by TIGR at http://www.tigr.org/cgi-binBlastSearch/blast.cgi
   Secreted proteins of this category are (the name of the homologous gene in *C*. *trachomatis* serovar D genome is given in parenthesis):
   CPn0105 (CT016), CPn0287, CPn0330 (CT083), CPn0334 (CT079), CPn0374 (CT056), CPn0379 (CT053), CPn0705 (CT671), CPn0710 (CT666), CPn0711 (CT665), CPn0820 (CT567), 0821 (CT566), CPn1016 (CT858), CPn1022 (CT863)

Moreover, the inventors have shown that CPn0175, expressed as a full-length protein with a carboxyl-terminal histidine tag, was secreted by a type III dependent mechanism *S*. *flexneri ipaB* strain. This is the first demonstration of the secretion of a full-length chlamydial protein by a type III apparatus.

Finally, the inventors have constructed chimeras with 13 genes from *C*. *trachomatis genome*. We found that 7 of these chimeras were secreted. The corresponding genes in *C*. *trachomatis* serovar D genome are CT053, CT083, CT529, CT665, CT666, CT671, CT863.

Transformed *ipaB* colonies expressing a CPn/cya chimera were isolated on plates containing the Congo Red dye. Only red colonies, indicative of a constitutive high level of type III secretion, were considered. Such colonies were picked on a LB plate and incubated for 6 hours at 37 C. A polyvinylidene fluoride membrane (Immobilon-P, Millipore) was deposited briefly incubated in ethanol and then processed for Western blotting using anti-cylase antibody described (Subtil et al (2001) Mol. Microbiol. 39:792-800). Revelation was done by enhanced chemifluorescence (Amersham). The signal for colonies in which the CPn/cya chimeras were not secreted was restricted to the area of the membrane that had been in contact with the colonies. The signal for colonies in which the CPn/cya chimeras were secreted appeared as a halo around the area of the membrane that had been in contact with the colonies.

Obviously, numerous modifications and variations on the present invention are possible in light of the above teachings. It is therefore, to be understood that within the scope of the appended claims, the invention may be practiced otherwise than as specifically described herein.

Unless otherwise defined, all technical and scientific, terms used herein have the same meaning as commonly understood by one of ordinary skill in the art of molecular biology. Although methods and materials similar or equivalent to those described herein can be used in the practice or testing of the present invention, suitable methods and materials are described herein. In case of conflict, the present specification, including definitions, will control. In addition, the materials, methods, and examples are illustrative only and are not intended to be limiting.

## Claims

1. A method for identifying a polypeptide of *Chlamydia* secreted by the type III secretion pathway, **characterized in that** it comprises the steps of:
(a) providing a recombinant expression vector comprising a DNA encoding at least a polypeptide of *Chlamydia*;
(b) transforming a *Shigella* strain containing a type III secretion pathway with said recombinant vector;
(c) expressing said vector in the *Shigella* strain transformed in (b); and
(d) detecting the secretion of said DNA expression product; wherein the secretion of said expression product indicates that it corresponds to a *Chlamydia* polypeptide secreted by the type III secretion pathway.

2. A method for identifying a polypeptide of *Chlamydia* secreted by the type III secretion pathway, **characterized in that** it comprises the steps of:
(a) providing a recombinant expression vector comprising a DNA encoding at least the N-terminal part of a *Chlamydia* polypeptide fused to a reporter gene;
(b) transforming a *Shigella* strain containing a type III secretion pathway with said recombinant vector;
(c) expressing said vector in the *Shigella* strain transformed in (b); and
(d) detecting the secretion of said reporter gene expression product; wherein the secretion of said expression product indicates that the fused DNA contains at least a polynucleotide that corresponds to a *Chlamydia* polypeptide secreted by the type III secretion pathway.

3. The method according to claim 1 or claim 2, **characterized in that** said reporter gene is a gene encoding a calcium-dependant adenylate cyclase (cya).

4. At method for screening an active molecule inhibiting the secretion of a secreted *Chlamydia* polypeptide by the type III secretion pathway, **characterized in that** it comprises the steps of:
(a) providing a recombinant expression vector containing a DNA encoding at least a polypeptide of *Chlamydia*, the secretion of which is to be inhibited;
(b) transforming a *Shigella* strain containing a type III secretion pathway with said recombinant vector;
(c) expressing said DNA of said vector in said transformed *Shigella* strain in the presence of a molecule to be tested;
(d) expressing said DNA of said vector in said transformed *Shigella* strain in the absence of the molecule to be tested; and
(e) comparing secretion of the DNA expression product of step (c) and step (d); wherein a decrease of said secretion is indicative of the ability of said tested molecule to inhibit secretion of said secreted *Chlamydia* polypeptide.

5. The method according to claims 1 to 4, **characterized in that** said polypeptide secreted by the type III secretion pathway is selected from the group consisting of IncA, Cpn0026, Cpn0308 and Cpn0585 of *Chlamydia pneumoniae*.

6. A method for screening a molecule which inhibits secretion of a *Chlamydia* polypeptide secreted by the type III secretion pathway, **characterized in that** it comprises:
(a) providing a recombinant expression vector containing a DNA encoding at least the N-terminal part of a polypeptide of *Chlamydia,* the secretion of said polypeptide being to be inhibited, fused to a reporter gene;
(b) transforming a *Shigella* strain containing a type III secretion pathway with said recombinant vector;
(c) expressing said vector in said transformed *Shigella* strain in the presence of a molecule to be tested;
(d) expressing said vector in said transformed *Shigella* strain in the absence of the molecule to be tested; and
(e) comparing secretion of the expression product of said reporter gene in step (c) and step (d); wherein a decrease of said secretion is indicative of the ability of said tested molecule to inhibit secretion of said secreted *Chlamydia* polypeptide.

7. The method according to claim 6, **characterized in that** said polypeptide secreted by the type III secretion pathway is selected from the group consisting of IncA, Cpn0026, Cpn0308 and Cpn0585 of *Chlamydia pneumoniae*.

8. The method according to any one of claims 4 to 7, **characterized in that** said reporter gene is a gene encoding a calcium dependant adenylate cyclase (cya).

## Patentansprüche

1. Verfahren zum Identifizieren eines Polypeptids von *Chlamydia*, das über den Typ III-Sekretionsweg sekretiert wird, **dadurch gekennzeichnet, dass** es die Stufen umfasst:
(a) Bereitstellen eines rekombinanten Expressionsvektors, der eine DNA, die für wenigstens ein Polypeptid von *Chlamydia* codiert, umfasst;
(b) Transformieren eines *Shigella*-Stammes, der einen Typ III-Sekretionsweg enthält, mit dem rekombinanten Vektor;
(c) Exprimieren des Vektors in dem in (b) transformierten *Shigella*-Stamm und
(d) Detektieren der Sekretion des DNA-Expressionsproduktes; wobei die Sekretion des Expressionsproduktes anzeigt, dass es einem *Chlamydia*-Polypeptid entspricht, das über den Typ III-Sekretionsweg sekretiert wird.

2. Verfahren zum Identifizieren eines Polypeptids von *Chlamydia*, das über den Typ III-Sekretionsweg sekretiert wird, **dadurch gekennzeichnet, dass** es die Stufen umfasst:
(a) Bereitstellen eines rekombinanten Expressionsvektors, der eine DNA umfasst, die für wenigstens den N-terminalen Teil eines *Chlamydia*-Polypeptids codiert, fusioniert mit einem Reportergen;
(b) Transformieren eines *Shigella*-Stammes, der einen Typ III-Sekretionsweg enthält, mit dem rekombinanten Vektor;
(c) Exprimieren des Vektors in dem in (b) transformierten *Shigella*-Stamm und
(d) Detektieren der Sekretion des Reportergen-Expressionsprodukts; wobei die Sekretion des Expressionsprodukts anzeigt, dass die fusionierte DNA wenigstens ein Polynukleotid enthält, das einem *Chlamydia*-Polypeptid entspricht, das über den Typ III-Sekretionsweg sekretiert wird.

3. Verfahren nach Anspruch 1 oder Anspruch 2, **dadurch gekennzeichnet, dass** das Reportergen ein Gen ist, das für eine calciumabhängige Adenylatcyclase (cya) codiert.

4. Verfahren zum Screening eines aktiven Moleküls, das die Sekretion eines sekretierten *Chlamydia*-Polypeptids über den Typ III-Sekretionsweg inhibiert, **dadurch gekennzeichnet, dass** es die Stufen umfasst:
(a) Bereitstellen eines rekombinanten Expressionsvektors, der eine DNA, die für wenigstens ein Polypeptid von *Chlamydia*, dessen Sekretion inhibiert werden soll, codiert, enthält;
(b) Transformieren eines *Shigella*-Stammes, der einen Typ III-Sekretionsweg enthält, mit dem rekombinanten Vektor;
(c) Exprimieren der DNA des Vektors in dem transformierten *Shigella*-Stamm in Gegenwart eines Moleküls, das getestet werden soll;
(d) Exprimieren der DNA des Vektors in dem transformierten *Shigella*-Stamm in Abwesenheit des Moleküls, das getestet werden soll; und
(e) Vergleichen der Sekretion des DNA-Expressionsprodukts von Stufe (c) und Stufe (d); wobei eine Abnahme der Sekretion anzeigend ist für die Fähigkeit des getesteten Moleküls, die Sekretion des sekretierten *Chlamydia*-Polypeptids zu inhibieren.

5. Verfahren nach den Ansprüchen 1 bis 4, **dadurch gekennzeichnet, dass** das Polypeptid, das über den Typ III-Sekretionsweg sekretiert wird, ausgewählt ist aus der Gruppe, bestehend aus IncA, Cpn0026, Cpn0308 und Cpn0585 von *Chlamydia pneumoniae*.

6. Verfahren zum Screening eines Moleküls, das die Sekretion eines *Chlamydia*-Polypeptids, das über den Typ III-Sekretionsweg sekretiert wird, inhibiert, **dadurch gekennzeichnet, dass** es umfasst:
(a) Bereitstellen eines rekombinanten Expressionsvektors, der eine DNA enthält, die für wenigstens den N-terminalen Teil eines Polypeptids von *Chlamydia* codiert, wobei die Sekretion des Polypeptids inhibiert werden soll, fusioniert mit einem Reportergen;
(b) Transformieren eines *Shigella*-Stammes, der einen Typ III-Sekretionsweg enthält, mit dem rekombinanten Vektor;
(c) Exprimieren des Vektors in dem transformierten *Shigella*-Stamm in Gegenwart eines Moleküls, das getestet werden soll;
(d) Exprimieren des Vektors in den transformierten *Shigella*-Stamm in Abwesenheit des Moleküls, das getestet werden soll; und
(e) Vergleichen der Sekretion des Expressionsprodukts des Reportergens in Stufe (c) und Stufe (d); wobei eine Abnahme der Sekretion anzeigend ist für die Fähigkeit des getesteten Moleküls, die Sekretion des sekretierten *Chlamydia-*Polypeptids zu inhibieren.

7. Verfahren nach Anspruch 6, **dadurch gekennzeichnet, dass** das Polypeptid, das über den Typ III-Sekretionsweg sekretiert wird, ausgewählt ist aus der Gruppe, bestehend aus IncA, Cpn0026, Cpn0308 und Cpn0585 von *Chlamydia pneumoniae.*

8. Verfahren nach einem der Ansprüche 4 bis 7, **dadurch gekennzeichnet, dass** das Reportergen ein Gen ist, das für eine calciumabhängige Adenylatcyclase (cya) codiert.

## Revendications

1. Méthode d'identification d'un polypeptide de *Chlamydia* sécrété par la voie de sécrétion de type III, **caractérisée en ce qu'**elle comprend les étapes consistant à :
(a) obtenir un vecteur d'expression recombinant comprenant un ADN codant au moins pour un polypeptide de *Chlamydia ;*
(b) transformer une souche de *Shigella* contenant une voie de sécrétion de type III avec ledit vecteur recombinant ;
(c) exprimer ledit vecteur dans la souche de *Shigella* transformée en (b) ; et
(d) détecter la sécrétion du produit d'expression dudit ADN; la sécrétion dudit produit d'expression indiquant qu'il correspond à un polypeptide de *Chlamydia* sécrété par la voie de sécrétion de type III.

2. Méthode d'identification d'un polypeptide de *Chlamydia* sécrété par la voie de sécrétion de type III, **caractérisée en ce qu'**elle comprend les étapes consistant à :
(a) obtenir un vecteur d'expression recombinant comprenant un ADN codant au moins pour la partie N-terminale d'un polypeptide de *Chlamydia* fusionné à un gène rapporteur ;
(b) transformer une souche de *Shigella* contenant une voie de sécrétion de type III avec ledit vecteur recombinant ;
(c) exprimer ledit vecteur dans la souche de *Shigella* transformée en (b) ; et
(d) détecter la sécrétion du produit d'expression dudit gène rapporteur ; la sécrétion dudit produit d'expression indiquant que l'ADN fusionné contient au moins un polynucléotide qui correspond à un polypeptide de *Chlamydia* sécrété par la voie de sécrétion de type III.

3. Méthode selon la revendication 1 ou la revendication 2, **caractérisée en ce \que** ledit gène rapporteur est un gène codant pour une adénylate cyclase (cya) calcium-dépendante.

4. Méthode de criblage d'une molécule active inhibant la sécrétion d'un polypeptide de *Chlamydia* sécrété par la voie de sécrétion de type III, **caractérisée en ce qu'**elle comprend les étapes consistant à :
(a) obtenir un vecteur d'expression recombinant contenant un ADN codant au moins pour un polypeptide de *Chlamydia,* dont la sécrétion est à inhiber ;
(b) transformer une souche de *Shigella* contenant une voie de sécrétion de type III avec ledit vecteur recombinant ;
(c) exprimer ledit ADN dudit vecteur dans ladite souche de *Shigella* transformée en présence d'une molécule à tester ;
(d) exprimer ledit ADN dudit vecteur dans ladite souche de *Shigella* transformée en l'absence de la molécule à tester ; et
(e) comparer la sécrétion du produit d'expression de l'ADN de l'étape (c) et de l'étape (d) ; une baisse de ladite sécrétion étant indicatrice de l'aptitude de ladite molécule testée à inhiber la sécrétion dudit polypeptide de *Chlamydia sécrété*.

5. Méthode selon les revendications 1 à 4, **caractérisée en ce que** ledit polypeptide sécrété par la voie de sécrétion de type III est choisi dans le groupe constitué d'IncA, de Cpn0026, de Cpn0308 et de Cpn0585 de *Chlamydia pneumoniae*.

6. Méthode de criblage d'une molécule qui inhibe la sécrétion d'un polypeptide de *Chlamydia* sécrété par la voie de sécrétion de type III, **caractérisée en ce qu'**elle comprend les étapes consistant à :
(a) fournir un vecteur d'expression recombinant contenant un ADN codant au moins pour la partie N-terminale d'un polypeptide de *Chlamydia,* la sécrétion dudit polypeptide étant à inhiber, fusionné à un gène rapporteur ;
(b) transformer une souche de *Shigella* contenant une voie de sécrétion de type III avec ledit vecteur recombinant ;
(c) exprimer ledit vecteur dans ladite souche de *Shigella* transformée en présence d'une molécule à tester ;
(d) exprimer ledit vecteur dans ladite souche de *Shigella* transformée en l'absence de la molécule à tester ; et
(e) comparer la sécrétion du produit d'expression dudit gène rapporteur dans l'étape (c) et dans l'étape (d) ; une baisse de ladite sécrétion étant indicatrice de l'aptitude de ladite molécule testée à inhiber la sécrétion dudit polypeptide de *Chlamydia sécrété.*

7. Méthode selon la revendication 6, **caractérisée en ce que** ledit polypeptide sécrété par la voie de sécrétion de type III est choisi dans le groupe constitué d'IncA, de Cpn0026, de Cpn0308 et de Cpn0585 de *Chlamydia pneumoniae*.

8. Méthode selon l'une quelconque des revendications 4 à 7, **caractérisée en ce que** ledit gène rapporteur est un gène codant pour une adénylate cyclase (cya) calcium-dépendante.
